# EUROPÄISCHE PATENTSCHRIFT

(11) **EP 2 060 287 B1**
(45) Veröffentlichungstag und Bekanntmachung des Hinweises auf die Patenterteilung: **15.06.2011**
(21) Anmeldenummer: 07120689.0
(22) Anmeldetag: 14.11.2007
(51) Int. Cl.: A61M 5/145, A61M 31/00

(54) **Medikamentenzuführungssystem für CED (Convection Enhanced Delivery)-Katheterinfusionen**
Drug supply system for CED (Convection Enhanced Delivery) catheter infusions
Système de délivrance de médicaments pour perfusions à cathéters CED (Convection enhanced delivery)

(43) Veröffentlichungstag der Anmeldung: 20.05.2009
(73) Patentinhaber: BrainLAB AG, 85622 Feldkirchen (DE)
(72) Erfinder: Mittermeyer, Stephan, 84036, Landshut (DE)
(74) Vertreter: Schwabe - Sandmair - Marx

(56) Entgegenhaltungen:
- WO-A-95/15191
- WO-A-2004/093945
- WO-A-2006/096286
- WO-A-2007/062068

## Beschreibung

Die Erfindung betrifft ein Medikainentenzuführungssystem für CED (Convection Enhanced Delivery)-Katheterinfusionen.

Die Verabreichung von Medikamenten im Rahmen der "Convection Enhanced Delivery (CED)" ist eine neurochirurgische Anwendung, bei welcher ein Katheter in festes Gehimgewebe eingesetzt werden muss. Durch den Katheter hindurch wird sehr langsam ein Medikament in das Gehirngewebe verabreicht. Zur Förderung des Medikaments ist eine Spritze vorgesehen, wobei unter diesem Begriff hierin ganz allgemein alle Vorrichtungen zu verstehen sind, die ein Medikament (zum Beispiel durch einen Kolben) aus einem Depot ausbringen. Die Spritze umfasst eine Kolbenvorschub- oder Pumpeinrichtung oder ist mit einer solchen Einrichtung verbunden. Durch diese Einrichtung wird mechanisch und vorzugsweise automatisch eine gewisse Medikamentenmenge pro Zeitabschnitt in eine Katheterzuleitung eingespritzt, und meist wird dadurch eine konstante Strömungsrate über mehrere Tage eingestellt.

Herkömmliche CED-Medikainentenzuführungssysteme weisen also eine direkte Medikamentenleitung von der Spritze zum Katheter auf; der Förderdruck wird durch die Fördereinrichtung (Spritze) direkt auf das Medikament übertragen.

Das Problem mit solchen herkömmlichen Zuführungssystemen besteht darin, dass das Medikamenten-Fluidsystem geöffnet werden muss, wenn die Zuführung manipuliert werden soll. Manipulationen sind aber manchmal notwendig, beispielsweise wenn eine neue, nachgefüllte Spritze angeschlossen werden soll, oder wenn der Patient vom Zuführungssystem getrennt werden muss, um beispielsweise einem CT- oder MR-Scan unterzogen zu werden. Beim Öffnen des Medikamenten-Fluidsystems kann es zu Kontaminierungen oder zum Eintritt von Luft in die Medikamentenleitungen kommen.

Es ist die Aufgabe der vorliegenden Erfindung, ein Medikamentenzuführungssystem für CED-Katheterinfusionen bereitzustellen, welches die vorgenannten Probleme löst. Insbesondere soll die Handhabung des Medikamentenzuführungssystems bei notwendigen Manipulationen leichter und sicherer gemacht werden.

Aus der WO 2006/096286 A1 ist ein Medikamentenzuführungssystem gemäß dem Oberbegriff des Patentanspruchs 1 bekannt.

Diese Aufgabe wird durch ein Medikamentenzuführungssystem gemäß dem Anspruch 1 gelöst. Die Unteransprüche definieren bevorzugte Ausführungsformen der Erfindung.

Gemeinsam mit dem oben beschriebenen Stand der Technik weist das Medikamentenzuführungssystem gemäß der vorliegenden Erfindung ein Medikamentenführungssystem auf, das ein Medikamentendepot und eine Katheterzuleitung umfasst, sowie eine Fördereinrichtung, welche für die Förderung des Medikaments aus dem Medikamentendepot in die Katheterzuleitung sorgt. Die vorliegende Erfindung sieht zur Lösung der oben genannten Probleme vor, dass das Medikamentenführungssystem ein nach außen fluiddicht abgeschlossenes System ist, und dass zwischen der Fördereinrichtung und dem Medikamentenführungssystem ein Förderfluid-Förderungssystem angeordnet ist, welches einerseits an die Fördereinrichtung angeschlossen ist und andererseits ohne Fluid-Medikamenten-Kontakt über ein Medikamenten-Verdrängerelement mit dem Medikamentendepot in Verbindung steht.

Mit anderen Worten wird gemäß der vorliegenden Erfindung zwischen die Fördereinrichtung und die tatsächliche Medikamentenführung ein weiteres System als "Transportsystem" zwischengeschaltet. Wenn nunmehr Manipulationen notwendig sind, können diese am "Transportsystem" ausgeführt werden, d.h. am Förderfluid-Förderungssystem, ohne dass das nach außen fluiddicht abgeschlossene Medikamentensystem manipuliert werden muss. Muss man beispielsweise die Fördereinrichtung (Spritzeneinrichtung) vom System trennen, damit der Patient beweglich wird, so kann diese Trennung, und ebenfalls der nachträgliche Neuanschluss im Fördersystem stattfinden, ohne eine Kontaminierung des Medikamentensystems befürchten zu müssen.

Während einer CED-Infusion, die sich über mehrere Tage erstrecken kann, kann es aus verschiedenen weiteren Gründen notwendig sein, die Spritzeneinrichtung von der Infusionsleitung zu trennen. Einer dieser Gründe ist beispielsweise das Ersetzen eines leeren Medikamentendepots durch ein neues, gefülltes Depot; aber auch wegen des Infektionsrisikos und aus Gründen der zu gewährleistenden Medikamentenstabilität kann eine solche Manipulation notwendig werden. Damit die Fördereinrichtung (Spritze oder Pumpe) risikolos vom System abgetrennt werden kann, wird erfindungsgemäß das Medikamentendepot bereitgestellt, welches das Medikament umfasst. Damit das Medikament aus dem Depot ausgebracht werden kann, wird ein anderes Fluid in das Medikamentendepot gepumpt, wobei eine Vermischung der beiden Fluide verhindert wird. Hierzu steht das Medikamenten-Verdrängerelement zur Verfügung.

Bei der vorliegenden Erfindung weist das Medikament direkt kontaktierende Medikamenten-Verdrängerelement eine flexible, ballonartig aufblähbare, insbesondere elastische Membran auf, die vollständig im Medikamentendepot untergebracht ist und bei einer Ausdehnung das Medikament aus dem Depot in das Medikamentenführungssystem hinein verdrängt.

Gemäß einem nicht zur Erfindung gehörigen Beispiel weist das Medikamenten-Verdrängerelement eine verschiebbare Wand im oder am Medikamentendepot auf, die vom Förderfluid durch Anpressdruck verschoben wird und dabei das Medikament aus dem Depot in das Medikamentenführungssystem hinein verdrängt.

Das Förderfluid-Förderungssystem kann ein Leitungssystem umfassen, das eine Trenn- und Anschlussstelle aufweist, insbesondere eine Schnellverbindung. Hierdurch wird ein leichtes Abtrennen im Fördersystem ermöglicht; ebenso ein schnelles Wiederanschließen.

Außerdem besteht die Möglichkeit, in einem Förderfluid-Förderungssystem mit Leitungssystem mehrere Hilfseinrichtungen unterzubringen, wie zum Beispiel einen Drei-Wege-Verteiler, insbesondere mit Drei-Wege-Verschluss- bzw. Durchgangshahn, und/oder ein Rückschlagventil.

Bei einer Ausführungsvariante weist das erfindungsgemäße Medikamentenzuführungssystem ein in mehrere Kammern unterteiltes Medikamentendepot oder mehrere Medikamentendepots auf, wobei jede Kammer bzw. jedes Depot eine eigene Förderfluid-Zuleitung hat. Dabei können die Förderfluid-Zuleitungen umschaltbar einzeln oder miteinander durch die Förderfluid-Fördereinrichtung mit Förderfluid beaufschlagt werden.

Wie schon oben kurz erwähnt, kann die Förderfluid-Fördereinrichtung eine mechanisch beaufschlagte Spritze sein, es besteht aber auch die Möglichkeit, jedwede andere Pumpe oder pumpenartige Einrichtung zu verwenden.

In weiterer Ausgestaltung wird für das erfindungsgemäße Medikamentenzuführungssystem eine Medikamentenzuführungs-Steuerung bzw. -Regelung bereitgestellt, welche die Verabreichung des Medikaments steuert bzw. regelt. Insbesondere kann eine solche Steuerung bzw. Regelung in Abhängigkeit von gemessenen äußeren Parametern, wie Zeitablauf, Temperatur oder bestimmten Konzentrationen chemischer Elemente bzw. Verbindungen erfolgen. Um solche Messdaten zu erhalten, würden in diesem Fall die geeigneten Messinstrumente an geeignet vorgesehenen Stellen angebracht und mit dem Antrieb des Zuführungssystems bzw. der Fördereinrichtung wird die Steuerungs- bzw. Regelungseinrichtung verbunden.

Die Erfindung wird im Weiteren anhand von Ausführungsformen näher erläutert. Sie kann alle hierin beschriebenen Merkmale einzeln oder in jedweder sinnvollen Kombination umfassen. In den beiliegenden Zeichnungen zeigen:
- Figur 1: ein erfindungsgemäßes Medikamentenzuführungssystem gemäß einer ersten Ausführungsform;
- Figur 2: ein System gemäß einem nicht zur Erfindung gehörigen Beispiel;
- Figur 3: ein erfindungsgemäßes System gemäß einer dritten Ausführungsform; und
- Figur 4: ein erfindungsgemäßes System gemäß einer vierten Ausführungsform.

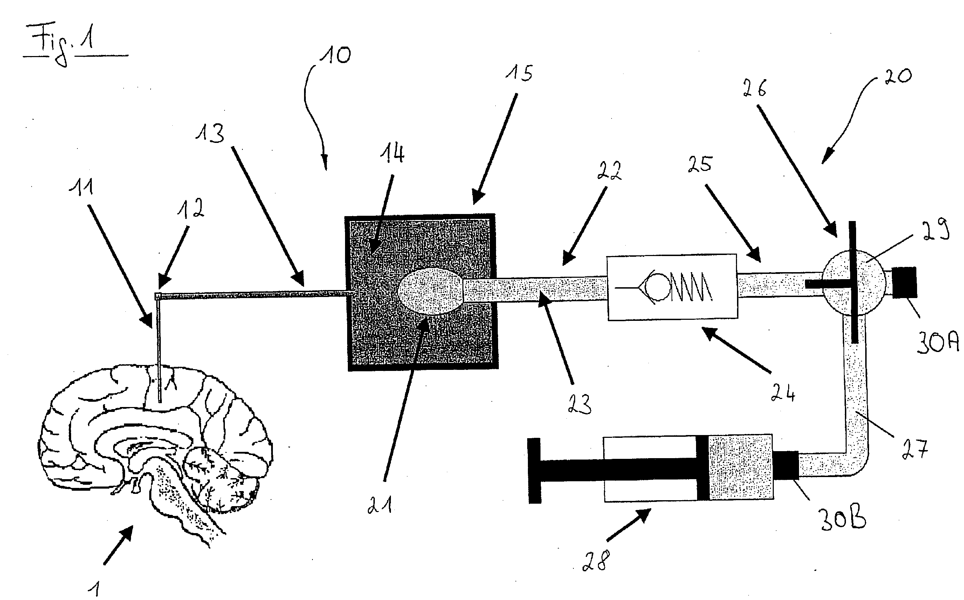

Die in Figur 1 dargestellte Ausführungsform eines erfindungsgemäßen Medikamentenzuführungssystems zeigt dieses System in seiner Funktionsumgebung. Das System dient dazu, ein Medikament über einen Katheter 11 in das feste Gewebe des links dargestellten Gehirns 1 einzubringen. Hierzu ist der Katheter 11 am Anschlusspunkt 12 mit einer Medikamentenleitung 13 verbunden, die vom Medikamentendepot 15 ausgeht und das Medikament 14 zum Anschlusspunkt 12 und in den Katheter 11 leitet. Der Systemteil vom Katheter 11 bis zum Depot 15, in dem lediglich das Medikament fließt, wird auch als Medikamentenführungssystem 10 bezeichnet. Zwischen der Spritze 28 und dem Medikamentendepot 15 befindet sich der andere Systemteil, nämlich das Förderfluid-Förderungssystem 20 mit der Leitung 27, dem Drei-Wege-Hahn 26 im Drei-Wege-Verteiler 29, der Leitung 25, dem Rückschlagventil 24, der Leitung 22 und dem Verdrängerkörper 21, der im Depot 15 untergebracht ist. Im Förderfluid-Förderungssystem 20 ist das Förderfluid 23, beispielsweise eine Salzlösung untergebracht. Schnellanschlüsse sind mit 30A und 30B bezeichnet.

Wenn nun die Spritze 28 gewechselt werden muss, wird der Drei-Wege-Hahn 26 geschlossen, und die Spritze kann abgenommen werden, ohne dass ein Druck im System verloren geht. Das Anschließen einer vorgefüllten Spritze an ein vorgefülltes System ist oftmals mit Druckspitzen und dem Einbringen von Luftblasen in die Leitung verbunden. Da das Förderfluid-System aber im vorliegenden Fall nicht direkt mit dem Medikamentensystem verbunden ist, wird die Luft nicht an das Medikamentensystem weitergeleitet und kann auch nicht durch den Katheter in das Gehirn eindringen. Damit wird die Rückflussproblematik an der Katheterspitze und eine nicht voraussagbare Medikamentenverteilung vermieden. Während die Infusion durchgeführt wird, strömt das Förderfluid 23 in den Verdrängerkörper 21, der hier ballonartig und aus einem flexiblen Material aufgebaut ist. Der Verdrängerkörper wird sich aufweiten und das Medikament 14 aus dem Depot 15 hinaus durch die Leitung 13 in den Katheter 11 und damit in das Gehirn bringen.

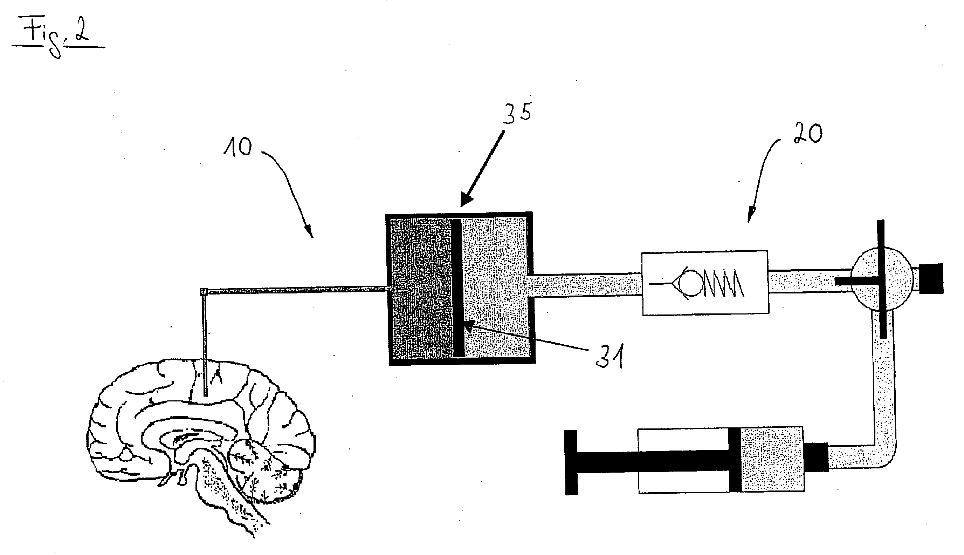

Beim in der Figur 2 gezeigten Bespiel ist das Depot 35 unterteilt, und zwar durch eine verschiebliche Membran 31, die das Medikamentenführungssystem 10 und das Förderfluid-Förderungssystem 20 teilt. Die Membran 31 verhindert einen Kontakt zwischen dem Förderfluid und dem Medikament. In der Zeichnung reicht sie nur deshalb nicht ganz an die Medikamentenwand heran, um ihre Verschieblichkeit darzustellen.

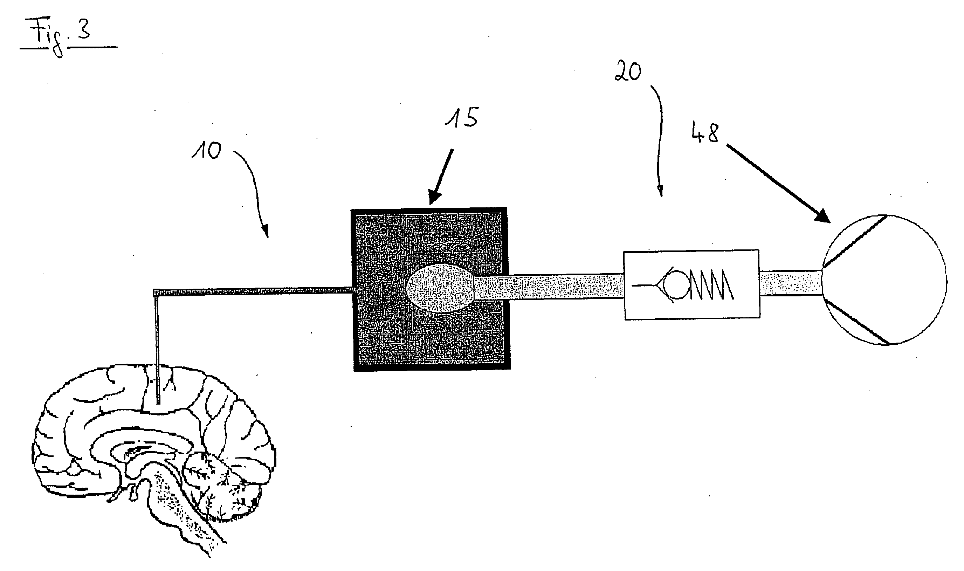

Das Förderfluid schiebt die Membran 31 nach links und bringt damit das Medikament zur Infusionsstelle. Bei einer anderen Ausführungsform, wie sie beispielsweise in Figur 3 dargestellt ist, kann die Spritze oder Spritzenpumpe durch jedwede andere Art von Infusionspumpe ersetzt werden, und eine solche Infusionspumpe ist in Figur 3 schematisch dargestellt und mit dem Bezugszeichen 48 versehen. Außerdem können druckbetriebene Pumpen anstelle von Volumenpumpen verwendet werden, da dies einen vorteilhaften Einfluss auf die Medikamentenverteilung hat.

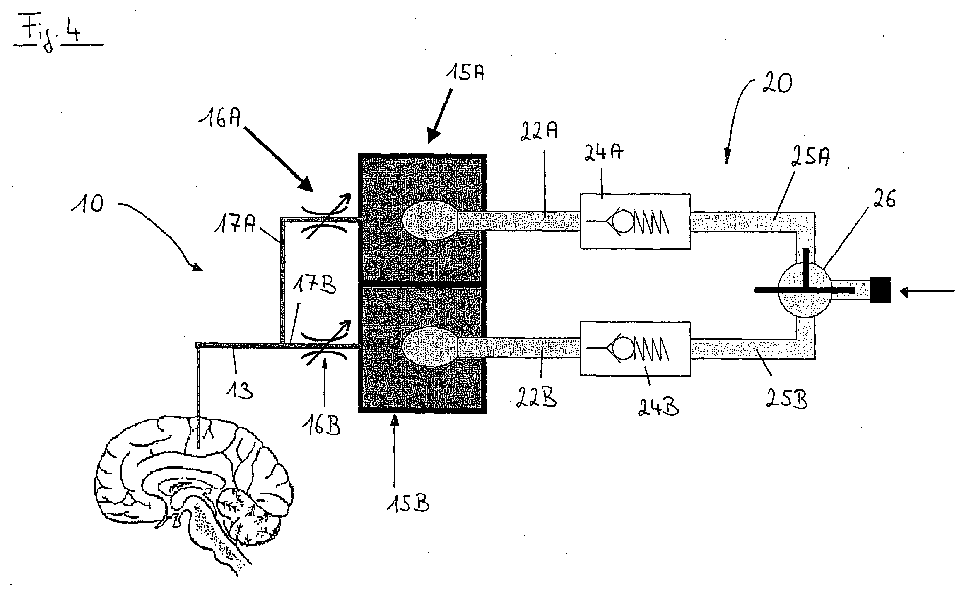

Bei einer anderen Ausführungsform, wie sie beispielsweise in Figur 4 dargestellt ist, hat das Medikamentendepot mehr als eine Kammer, in der Figur 4 die beiden Kammern bzw. einzelnen Depots 15A und 15B. Die Vorrichtung weist dann zwei Förderfluid-Förderungssysteme auf, deren Elemente entsprechend der Figur 1 benannt sind, wobei sie jeweils die Bezeichnungen A bzw. B tragen. Das Drei-Wege-Ventil 26 erlaubt die Umschaltung, Zuschaltung oder Abschaltung der einzelnen Leitungsabschnitte A, B. Eine solche Ausführungsform kann beispielsweise verwendet werden, wenn unterschiedliche Medikamente verabreicht werden sollen oder - bei chronischen Anwendungen -, um ein Depot wieder auffüllen zu können, während das andere die Infusion weiterführt.

Das Medikamentendepot kann auch eine spezifische Umgebung bereitstellen, wenn dies notwendig ist. Beispielsweise kann das Medikament am Depot gekühlt werden, und es ist erfmdungsgemäß dann nicht mehr nötig, auch das gesamte Fördersystem zu kühlen. Das gesamte System oder nur das Medikamenten-Zuführungssystem können als subkutanes (implantierbares) System ausgestaltet werden.

Zusätzlich können, wie in Figur 4 gezeigt ist, an den Medikamenten-Ausgangsleitungen 17A und 17B Drosselventile 16A und 16B bereitgestellt werden. Die Drosselventile 16A und 16B können durch äußere Parameter angesteuert werden, um die Medikamentenverabreichung an solche Parameter anzupassen. Die Parameter können beispielsweise Zeitablauf aber auch die Konzentration von chemischen Elementen oder Verbindungen sein, die durch einen Sensor gemessen werden.

Das Trennen des mit dem Medikament befüllten Systems von dem System, das mit dem Förderfluid gefüllt wird, bietet verschiedene Vorteile, die hier nochmals aufgeführt werden sollen. Es verhindert die Einbringung von Luft in die Medikamenten-Infusionsleitung. Wenn das Medikamentensystem einmal mit dem Medikament vorgefüllt ist (geprimed) und die Luft vollständig aus dem Medikamentensystem entfernt wurde, wird keine zusätzliche Luft mehr in das System aufgrund von Leitungsabtrennungen oder Leitungsanbringungen bzw. der Abtrennung oder Neuanbringung von Spritzen eingebracht. Das Vermeiden des Eindringens von Luft in die Infusionsleitung ist für das Ergebnis der Behandlung sehr kritisch, da jede Luftblase in der Infusionsleitung oder im Gewebe den Rückfluss des Medikaments entlang des Katheters verstärkt, was zu einer Leckage und zu einer ungenügenden Behandlung des Patienten führt. Zusätzliche Luft führt außerdem zu einer chaotischen Medikamentenverteilung, die nicht vorhersehbar ist.

Ein System, das es gestattet, die Spritze oder Pumpe von der Infusionsleitung zu trennen, macht verschiedene Prozeduren sicherer und effizienter. Die Möglichkeit, die Spritzen zu wechseln, gestattet beispielsweise die Verwendung von Spritzen mit einem geringeren Volumen, was einen positiven Effekt auf die Rückflusslänge hat. Je größer das Spritzenvolumen ist, desto höher sind die Druck-Fluktuationen in der Infusionsleitung aufgrund der Spritzen-Pumptätigkeit.

Ferner wird es möglich, eine Infusion zu unterbrechen, wenn beispielsweise ein MR-Scan gemacht werden muss. Um einen solchen Scan durchzuführen, würden die Pumpen MRkompatibel sein müssen, was sie sehr teuer in der Herstellung macht, oder man muss eine sehr lange Verlängerungsleitung verwenden, was wiederum zu Druck-Fluktuationen in der Infusionsleitung führt.

Derzeit werden Katheter oft mit einer Salzlösung vorgefüllt (geprimed) und dann mit der Spritze verbunden, die das Medikament enthält. Dies beeinflusst offensichtlicher Weise die Konzentration des Medikaments, und die Trennung des Medikaments von der Förderlösung umgeht dieses Problem.

Das Infektionsrisiko kann bei einem geschlossenen Medikamentensystem signifikant verringert werden. Außerdem kann beispielsweise das Medikamentendepot am Körper getragen werden (zum Beispiel am Gürtel), und es besteht dann die Möglichkeit, unterschiedliche Verlängerungsleitungen mit unterschiedlichen Längen zu nutzen (beispielsweise eine längere Leitung, wenn der Patient sich bewegen möchte). Zusätzlich können diese Verlängerungsleitungen für das Förderfluid starrer ausgestaltet werden, da Bewegungen der Verlängerungsleitung nicht direkt in Bewegungen der Katheterröhre resultieren. Starre Röhren bzw. Leitungen haben geringere Druckschwankungen.

Ein weiterer Vorteil besteht darin, dass das Volumen von nicht genutztem Medikament signifikant verringert werden kann, weil die langen Leitungen mit Förderfluid gefüllt sind, nicht mit dem Medikament. Die Medikamentenleitung kann relativ kurz gehalten werden, so dass nur wenig von dem oft sehr teuren Medikament in den Leitungen verbleibt, wenn die Infusion beendet wird.

## Patentansprüche

1. Medikamentenzuführungssystem für CED (Convection Enhanced Delivery)-Katheterinfusionen mit:
- einem Medikamentenführungssystem (10), das ein Medikamentendepot (15) und eine Katheterzuleitung (13) umfasst, und
- einer Fördereinrichtung (28), welche für die Förderung des Medikaments (14.) aus dem Medikamentendepot (15) in die Katheterzuleitung (13) sorgt, wobei
- das Medikamentenführungssystem (10) ein nach außen fluiddicht abgeschlossenes System ist, und wobei
- zwischen der Fördereinrichtung (28) und dem Medikamentenführungssystem (10) ein Förderfluid-Förderungssystem (20) angeordnet ist, welches einerseits an die Fördereinrichtung (28) angeschlossen ist und andererseits ohne Fluid-Medikamenten-Kontakt über ein Medikamenten-Verdrängerelement (21) mit dem Medikamentendepot (15) in Verbindung steht, **dadurch gekennzeichnet, dass** das Medikamenten-Verdrängerelement eine flexible, das Medikament direkt kontaktiert und ballonartig aufblähbare, insbesondere elastische Membran (21) aufweist, die vollständig im Medikamentendepot (15) untergebracht ist und bei einer Ausdehnung das Medikament (14) aus dem Depot (15) in das Medikamentenführungssystem (10) hinein verdrängt.

2. Medikamentenzuführungssystem nach Anspruch 1, **dadurch gekennzeichnet, dass** das Förderfluid-Förderungssystem (20) ein Leitungssystem (22, 25, 27) umfasst, das eine Trenn- und Anschlussstelle aufweist, insbesondere eine Schnellverbindung.

3. Medikamentenzuführungssystem nach Anspruch 1 oder 2, **dadurch gekennzeichnet, dass** das Förderfluid-Förderungssystem (20) ein Leitungssystem (22, 25, 27) umfasst, wobei in dem Leitungssystem (22, 25, 27) ein Drei-Wege-Verteiler (29), insbesondere mit Drei-Wege-Verschluss- bzw. Durchgangshahn, und/oder ein Rückschlagventil (24) angeordnet ist bzw. sind.

4. Medikamentenzuführungssystem nach einem der Ansprüche 1 bis 3, **dadurch gekennzeichnet, dass** es ein in mehrere Kammern unterteiltes Medikamentendepot oder mehrere Medikamentendepots (15A, 15B) aufweist, wobei jede Kammer bzw. jedes Depot (15A, 15B) eine eigene Förderfluid-Zuleitung (22A, 22B) aufweist.

5. Medikamentenzuführungssystem nach Anspruch 4, **dadurch gekennzeichnet, dass** die Förderfluid-Zuleitungen (22A, 22B) umschaltbar einzeln oder miteinander durch die Förderfluid-Fördereinrichtung mit Förderfluid beaufschlagt werden.

6. Medikamentenzuführungssystem nach einem der Ansprüche 1 bis 5, **dadurch gekennzeichnet, dass** die Förderfluid-Fördereinrichtung eine mechanisch beaufschlagte Spritze oder eine Pumpe ist.

7. Medikamentenzuführungssystem nach einem der Ansprüche 1 bis 6, **dadurch gekennzeichnet, dass** es eine Medikamentenzuführungs-Steuerung bzw. Regelung aufweist, welche die Verabreichung des Medikaments steuert bzw. regelt, insbesondere in Abhängigkeit von gemessenen äußeren Parametern, wie Zeitablauf, Temperatur oder bestimmten Konzentrationen chemischer Elemente bzw. Verbindungen.

## Claims

1. A drug supply system for CED (convection-enhanced delivery) catheter infusions, comprising:
- a drug conducting system (10) which comprises a drug depot (15) and a catheter supply line (13); and
- a conveying device (28) which provides for conveying the drug (14) from the drug depot (15) into the catheter supply line (13); wherein
- the drug conducting system (10) is a system which is closed to the outside in a fluidic seal, and wherein
- a conveying fluid conveying system (20) is arranged between the conveying device (28) and the drug supply system (10) and is connected on the one hand to the conveying device (28) and on the other to the drug depot (15), via a drug displacement element (21), without fluid-drug contact, **characterised in that** the drug displacement element directly contacts the drug and comprises a flexible, in particular elastic membrane (21) which can be inflated like a balloon and is completely accommodated in the drug depot (15) and, when dilated, displaces the drug (14) from the depot (15) into the drug conducting system (10).

2. The drug supply system according to claim 1, **characterised in that** the conveying fluid conveying system (20) comprises a line system (22, 25, 27) which comprises a separating and connecting point, in particular a quick-release connection.

3. The drug supply system according to claim 1 or 2, **characterised in that** the conveying fluid conveying system (20) comprises a line system (22, 25, 27), wherein a three-way distributor (29), in particular comprising a three-way stop cock and/or straight-way cock, and/or a reflux valve (24) is/are arranged in the line system (22, 25, 27).

4. The drug supply system according to any one of claims 1 to 3, **characterised in that** it comprises a drug depot which is sub-divided into a number of chambers or comprises a number of drug depots (15A, 15B), wherein each chamber and/or depot (15A, 15B) comprises a conveying fluid supply line (22A, 22B) of its own.

5. The drug supply system according to claim 4, **characterised in that** the conveying fluid supply lines (22A, 22B) can be switched individually or together and charged with conveying fluid by the conveying fluid conveying device.

6. The drug supply system according to any one of claims 1 to 5, **characterised in that** the conveying fluid conveying device is a mechanically charged syringe or a pump.

7. The drug supply system according to any one of claims 1 to 6, **characterised in that** it comprises a drug supply controller and/or regulator which controls and/or regulates how the drug is administered, in particular in accordance with measured external parameters such as elapsed time, temperature or particular concentrations of chemical elements and/or compounds.

## Revendications

1. Système d'administration de médicaments pour des perfusions de type CED (Convection Enhanced Delivery) par cathéters, comportant :
- un système d'administration du médicament (10), qui comprend une réserve de médicament (15) et une conduite d'alimentation du cathéter (13), et
- un dispositif de transport (28), qui assure le transport du médicament (14) depuis la réserve de médicament (15) vers la conduite d'alimentation du cathéter (13),
- le système d'administration du médicament (10) étant un système fermé vers l'extérieur, de manière étanche aux fluides, et
- entre le dispositif de transport (28) et le système d'administration du médicament (10) étant disposé un dispositif de transport du fluide transporteur (20) qui, d'un côté, est raccordé au dispositif de transport (28) et, de l'autre côté, communique avec la réserve de médicament (15) sans entrer en contact avec le fluide et le médicament, par l'intermédiaire d'un élément de refoulement du médicament (21),
**caractérisé en ce que** l'élément de refoulement du médicament comporte une membrane (21), en particulier élastique, qui entre directement en contact avec le médicament et est propre à se déployer en forme de ballonnet et qui est intégralement logée dans la réserve de médicament (15) et, sous l'effet de son déploiement, pousse le médicament (14) hors de la réserve (15) vers l'intérieur du système d'administration du médicament (10).

2. Système d'administration de médicaments selon la revendication 1, **caractérisé en ce que** le dispositif de transport du fluide transporteur (20) comprend un système de conduites (22, 25, 27), qui comporte une zone de séparation et de raccordement, en particulier un raccord à assemblage rapide.

3. Système d'administration de médicaments selon la revendication 1 ou 2, **caractérisé en ce que** le dispositif de transport du fluide transporteur (20) comprend un système de conduites (22, 25, 27), sachant que dans ledit système de conduites (22, 25, 27) est ou sont monté(s) un ou des distributeurs à trois voies (29), en particulier avec un robinet de fermeture ou de passage à trois voies, et/ou un clapet anti-retour (24).

4. Système d'administration de médicaments selon l'une quelconque des revendications 1 à 3, **caractérisé en ce qu'**il comporte une réserve de médicament, divisée en plusieurs compartiments, ou plusieurs réserves de médicament (15A, 15B), chaque compartiment ou chaque réserve (15A, 15B) comportant sa propre conduite d'alimentation en fluide transporteur (22A, 22B).

5. Système d'administration de médicaments selon la revendication 4, **caractérisé en ce que** les conduites d'alimentation en fluide transporteur (22A, 22B) sont alimentées en fluide transporteur de manière réglable isolément ou conjointement par le dispositif de transport du fluide transporteur.

6. Système d'administration de médicaments selon l'une quelconque des revendications 1 à 5, **caractérisé en ce que** le dispositif de transport du fluide transporteur est une seringue sollicitée mécaniquement ou une pompe.

7. Système d'administration de médicaments selon l'une quelconque des revendications 1 à 6, **caractérisé en ce qu'**il comporte un dispositif de commande ou de réglage de l'administration du médicament, qui commande ou règle l'administration du médicament, en particulier en fonction des paramètres extérieurs mesurés, tels que le temps écoulé, la température ou des concentrations déterminées des éléments ou composés chimiques.
